(19) Europäisches Patentamt
European Patent Office
Office européen des brevets

(11) EP 3 744 255 B1

# (12) EUROPEAN PATENT SPECIFICATION

(45) Date of publication and mention of the grant of the patent:
**15.11.2023 Bulletin 2023/46**

(21) Application number: **19743956.5**

(22) Date of filing: **23.01.2019**

(51) International Patent Classification (IPC):
***A61B 5/22*** (2006.01) ***A61B 5/0245*** (2006.01)

(52) Cooperative Patent Classification (CPC):
**A61B 5/222; A61B 5/02438; A61B 5/0245; A61B 5/4866;** A61B 5/6823; A61B 5/6824; A61B 2503/10; A61B 2505/09

(86) International application number:
**PCT/JP2019/002000**

(87) International publication number:
**WO 2019/146616 (01.08.2019 Gazette 2019/31)**

## (54) EXERCISE LOAD ESTIMATION METHOD, EXERCISE LOAD ESTIMATION DEVICE, AND RECORDING MEDIUM

VERFAHREN ZUR SCHÄTZUNG DER TRAININGSBELASTUNG, VORRICHTUNG ZUR SCHÄTZUNG DER TRAININGSBELASTUNG UND AUFZEICHNUNGSMEDIUM

PROCÉDÉ D’ESTIMATION DE CHARGE D’EXERCICE, DISPOSITIF D’ESTIMATION DE CHARGE D’EXERCICE ET SUPPORT D’ENREGISTREMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2018 JP 2018009415**

(43) Date of publication of application:
**02.12.2020 Bulletin 2020/49**

(73) Proprietor: **NIPPON TELEGRAPH AND TELEPHONE CORPORATION**
**Chiyoda-ku, Tokyo 100-8116 (JP)**

(72) Inventors:
- **HIGUCHI, Yuichi**
**Musashino-shi, Tokyo 180-8585 (JP)**
- **ISHIHARA, Takako**
**Musashino-shi, Tokyo 180-8585 (JP)**
- **ONO, Kazuyoshi**
**Musashino-shi, Tokyo 180-8585 (JP)**

(74) Representative: **Samson & Partner Patentanwälte mbB**
**Widenmayerstraße 6**
**80538 München (DE)**

(56) References cited:
**CN-A- 106 730 763**
**JP-A- 2000 090 179**
**JP-A- 2004 261 608**
**JP-A- 2008 086 480**
**JP-A- 2008 220 498**
**JP-A- 2016 083 349**
**US-A1- 2009 287 103**
**US-A1- 2016 213 979**
**US-A1- 2016 354 636**

- **SHE JINHUA ET AL: "Selection of suitable maximum-heart-rate formulas for use with Karvonen formula to calculate exercise intensity", INTERNATIONAL JOURNAL OF AUTOMATION AND COMPUTING, ZHONGGUO KEXUE ZAZHISHE, CN, vol. 12, no. 1, 29 December 2014 (2014-12-29), pages 62-69, XP035988242, ISSN: 1476-8186, DOI: 10.1007/S11633-014-0824-3 [retrieved on 2014-12-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field

The present invention relates to an exercise load estimation method, an exercise load estimation device, and a recording medium and, more particularly, to a technique of estimating an exercise load using heartbeats.

### Background Art

Training is a daily activity for athletes. Safe and effective training is indispensable to athletes. If the load of training is insufficient for a target person such as an athlete who trains, even longtime training may not be effective. If the load of training is excessively high, the injury risk rises and it may become difficult to continue training. It is therefore important to grasp the state of the target person and set a training load appropriate for the purpose.

The rating of perceived exertion (RPE) has been conventionally known as an index for grasping the intensity of exercise such as training. RPE numerically represents a subjective degree of burden during exercise on a target person who exercises, such as an athlete.

An exercise load (to be referred to as a "workload" or "training load" hereinafter) obtained from the product of RPE and a time during which exercise was done has been conventionally used as an index for setting a proper load of exercise such as training (see, for example, non-patent literature 1). The product of RPE and a time during which exercise was done, for example, the length of a training session serving as a time during which training of a given event was done is also called session RPE (see non-patent literature 1).

### Related Art Literature

US 2009/0287103 A1 relates to monitoring and displaying patient activity and exercise and discloses subjectmatter according to the preamble of claim 1.

CN 106730763 relates to another example of monitoring exercise.

US 2016/0354636 A1 relates to providing exercise program based on feedback.

US 2016/0213979 A1 relates to providing exercise guide information.

### Non-Patent Literature

Non-Patent Literature 1: Gabbett, Tim J. "The training-injury prevention paradox: should athletes be training smarter and harder?." Br J Sports Med (2016): bjsports-2015.

Non-Patent Literature 2: https://ja.wikipedia.org/wiki/運動強度 (searched on January 5, 2018) She, Jinhua et al "Selection of Suitable Maximum-heart-rate Formulas for Use with Karvonen Formulate to Calculate exercise Intensity" provides further technical background.

### Disclosure of Invention

### Problem to be Solved by the Invention

However, a conventional workload obtained from the product of RPE and the time uses RPE based on the subjectivity of a target person. RPE needs to be reported from the target person, so correct information may not always be obtained. It is hard to obtain a workload as an objective index.

The present invention has been made to solve the above-described problems and has as its object to provide an exercise load estimation technique capable of estimating a workload from objective data.

### Means of Solution to the Problem

In order to achieve the above object of the present invention, there is provided an exercise load estimation method according to claim 1, an exercise load estimation device according to claim 3, and a computer readable recording medium according to claim 6.

Effect of the Invention

According to the present invention, a workload is calculated based on the heart rate of a target person who exercises, and the length of a measurement period in which the heart rate is measured. The workload of the target person can be estimated from objective data without using RPE or session RPE.

Brief Description of Drawings

Fig. 1 is a graph for explaining the principle of a workload estimation device according to the first embodiment of the present invention;
Fig. 2 is a block diagram showing the functional arrangement of the workload estimation device according to the first embodiment of the present invention;
Fig. 3 is a block diagram showing the hardware arrangement of the workload estimation device according to the first embodiment of the present invention;
Fig. 4 is a flowchart for explaining the operation of the workload estimation device according to the first embodiment of the present invention;
Fig. 5 is a graph for explaining an outline of a workload estimation device according to the second embodiment of the present invention;
Fig. 6 is a block diagram showing the functional arrangement of the workload estimation device according to the second embodiment of the present invention;
Fig. 7 is a flowchart for explaining the operation of the workload estimation device according to the second embodiment of the present invention;
Fig. 8 is a block diagram showing the functional arrangement of a workload estimation device according to the third embodiment of the present invention;
Fig. 9 is a flowchart for explaining the operation of the workload estimation device according to the third embodiment of the present invention;
Fig. 10 is a graph for explaining an outline of a workload estimation device according to the fourth embodiment of the present invention;
Fig. 11 is a block diagram showing the functional arrangement of a workload estimation device according to the fifth embodiment of the present invention;
Fig. 12 is a flowchart for explaining the operation of the workload estimation device according to the fifth embodiment of the present invention;
Fig. 13 is a block diagram showing the functional arrangement of a workload estimation device according to the sixth embodiment of the present invention; and
Fig. 14 is a flowchart for explaining the operation of the workload estimation device according to the sixth embodiment of the present invention.

Best Mode for Carrying Out the Invention

Preferred embodiments of the present invention will now be described in detail with reference to Figs. 1 to 14.

[General Description]

Fig. 1 is a graph for explaining the principle of a workload estimation device 1 according to the first embodiment. The abscissa of a graph shown in Fig. 1 represents a workload (predicted exercise load) obtained from the product of RPE and the time. The ordinate represents a value obtained from the product of the time and an exercise intensity calculated based on a maximum heart rate in a period in which a target person did exercise such as training.

The exercise intensity is a scale representing the severity of exercise based on the physical ability of a target person who exercises. Note that an exercise intensity calculated based on the maximum heart rate of the target person during exercise is especially called a "maximum exercise intensity".

RPE shown in Fig. 1 is a value obtained using a well-known modified Borg scale. The maximum heart rate of a target person during exercise used to calculate a maximum exercise intensity on the ordinate is converted into a maximum exercise intensity that takes a value of 0 to 10 so as to match the RPE range.

The maximum exercise intensity is calculated according to equation (1) (see non-patent literature 2):

$$\text{maximum exercise intensity} = (\text{measured maximum heart rate during exercise} - \text{resting heart rate})/(\text{maximum heart rate} - \text{resting heart rate}) \times 10$$

where "measured maximum heart rate during exercise" is a value measured by a heart rate monitor or the like, and "resting heart rate" and "maximum heart rate" are actually premeasured values.

As shown in Fig. 1, a RPE-based workload and a value based on the maximum heart rate of the target person during exercise are highly correlated to each other because the correlation coefficient R is close to 1. As described above, the slope of a regression line shown in Fig. 1 takes a value close to 1 in data in which the range of RPE and that of the maximum heart rate of the target person during exercise match each other.

This means that a value obtained from the product of RPE and the time, and a value obtained from the product of the time and a maximum exercise intensity based on the maximum heart rate of the target person during exercise are convertible. Thus, a value obtained from the product of the time and a maximum exercise intensity based on the maximum heart rate of the target person during exercise can be used as a workload, instead of a workload obtained from the product of RPE and the time. According to the present invention, a workload is estimated based on the heart rate of the target person during exercise.

[First Embodiment]

A workload estimation device 1 according to the first embodiment will be described in detail below.

Fig. 2 is a block diagram showing the functional arrangement of the workload estimation device 1 according to the first embodiment. The workload estimation device 1 includes a measurement unit 10, a storage unit 11, an extraction unit (first extraction unit) 12, an estimation unit 13, and an output unit 16.

The measurement unit 10 measures the heart rate of a target person in a period in which he/she exercises. The measurement unit 10 is implemented by a heart rate monitor or the like. The measurement unit 10 measures a beat rate per minute as a heart rate. A measurement period serving as a period in which a target person exercises can be arbitrary. However, a period (to be sometimes called a "session" hereinafter) divided by the event or genre of exercise such as training may also be used. For example, when the target person is a soccer player, one game of soccer can be one session. Note that the measurement unit 10 may measure the pulse rate of the target person instead of the heart rate.

The storage unit 11 stores the heart rate of the target person in the measurement period measured by the measurement unit 10. The storage unit 11 may store a pre-acquired RPE value of the target person and a workload value (predicted exercise load) obtained from the product of the RPE of the target person and the time. Also, the storage unit 11 stores an actually premeasured resting heart rate and maximum heart rate of the target person. The storage unit 11 stores information about the measurement period in which the measurement unit 10 measures a heart rate, for example, when the measurement period is one session, information about the time of the session.

The extraction unit 12 extracts a maximum heart rate in the measurement period from data of the heart rate of the target person measured by the measurement unit 10. The maximum heart rate is a value representing the limit value of a heart rate when the heart of the target person beats fastest during exercise. The extraction unit 12 stores the extracted maximum heart rate in the storage unit 11. The value of the maximum heart rate extracted by the extraction unit 12 is substituted into "measured maximum heart rate during exercise" in the above-described equation (1).

The estimation unit 13 includes an exercise intensity calculation unit 14 and a workload calculation unit (exercise load calculation unit) 15. The estimation unit 13 estimates the workload of the target person based on the heart rate of the target person measured by the measurement unit 10 and the length of the measurement period.

The exercise intensity calculation unit 14 calculates a maximum exercise intensity according to the above-described equation (1) based on the maximum heart rate of the target person extracted by the extraction unit 12. In equation (1), "resting heart rate" and "maximum heart rate" can be actually premeasured values. Alternatively, values calculated by subtracting the age of the target person from a "resting heart rate" value of 60 and a "maximum heart rate" value of 220 may be used respectively. The exercise intensity calculation unit 14 stores the calculated maximum exercise intensity in the storage unit 11.

The workload calculation unit 15 calculates a workload based on the length of the measurement period and the maximum exercise intensity of the target person calculated by the exercise intensity calculation unit 14. More specifically, the workload calculation unit 15 calculates a value by multiplying the length of the measurement period and the value of the maximum exercise intensity calculated by the exercise intensity calculation unit 14. The workload calculation unit 15 obtains the estimated value of the workload of the target person. When a training session is used as the measurement period, the workload of the target person is calculated for each session.

When a training session is used as the measurement period, the workload calculation unit 15 calculates a workload by multiplying a maximum heart rate in each session and the session time. In this case, a workload may be calculated by adding workloads of respective sessions.

The workload calculated by the workload calculation unit 15 is a value equivalent to a workload obtained from the product of RPE and the time mentioned above. The workload calculated by the workload calculation unit 15 can be used as an index for relatively analyzing the total load of training of a target person such as an athlete. For example, an athlete himself or a coach can more appropriately consider the recovery, adaptation, and the like of the athlete based on the workload, and set a training amount and the like in a predetermined period.

The output unit 16 outputs the workload value of the target person calculated by the workload calculation unit 15. The output unit 16 may display the calculated workload value on a display screen or the like in the workload estimation device 1.

[Hardware Arrangement of Workload Estimation Device]

Next, the hardware arrangement of the workload estimation device 1 having the above-described functional arrangement will be described with reference to the block diagram of Fig. 3.

As shown in Fig. 3, the workload estimation device 1 can be implemented by a computer including an arithmetic device 102 having a CPU 103 and a main storage device 104, a communication control device 105, a sensor 106, and an external storage device 107, which are connected via a bus 101, and programs for controlling these hardware resources.

The CPU 103 and the main storage device 104 constitute the arithmetic device 102. Programs for performing various control and arithmetic operations by the CPU 103 are stored in advance in the main storage device 104. The arithmetic device 102 implements the functions of the workload estimation device 1 such as the extraction unit 12, and the estimation unit 13 including the exercise intensity calculation unit 14 and the workload calculation unit 15 shown in Fig. 2.

The communication control device 105 is a control device for connecting the workload estimation device 1 and various external electronic devices via a communication network NW. The communication control device 105 may receive heart rate data via the communication network NW from the sensor 106 (to be described later) attached to a target person.

The sensor 106 is implemented by a heart rate monitor. The sensor 106 is attached to the chest, wrist, or the like of a target person while he/she exercises, and the sensor 106 measures his/her heart rate. For example, the sensor 106 attached to the chest measures an electrocardiogram using electrodes (not shown), detects heartbeats from a change of the electrocardiogram, and measures as a heart rate a beat rate per minute from the interval between heartbeats. As described above, the sensor 106 may be a pulsometer that measures the pulses of a target person.

The external storage device 107 is constituted by a readable/writable storage medium, and a driving device for reading and writing various kinds of information such as programs and data from and in the storage medium. The external storage device 107 can use as the storage medium a hard disk or a semiconductor memory such as a flash memory. The external storage device 107 can include a data storage 107a, a program storage 107b, and other storage devices (not shown) such as a storage device for backing up programs, data, and the like stored in the external storage device 107.

The heart rate of the target person measured by the sensor 106 is stored in the data storage 107a. The data storage 107a corresponds to the storage unit 11 shown in Fig. 2.

Various programs for executing processes necessary to estimate a workload, such as extraction processing, exercise intensity calculation processing, and workload calculation processing according to this embodiment are stored in the program storage 107b.

A timer 108 is the internal timer of the workload estimation device 1. A measurement period in which the heart rate of a target person is measured is determined based on time information obtained by the timer 108.

A display device 109 constitutes the display screen of the workload estimation device 1 and functions as the output unit 16 (to be described later). The display device 109 is implemented by a liquid crystal display or the like.

[Operation of Workload Estimation Device]

Next, the operation of the workload estimation device 1 having the above-described arrangement will be described with reference to the flowchart of Fig. 4. First, a target person wears on his/her chest, wrist, or the like the measurement unit 10 implemented by a heart rate monitor, and starts exercise such as training.

The measurement unit 10 measures the heart rate of the target person in a period in which he/she does exercise such as training (step S1). Data of the heart rate of the target person measured by the measurement unit 10 is stored in the storage unit 11. Information representing the length of the training time during which the measurement unit 10 performed measurement, that is, the measurement period is also stored in the storage unit 11. Note that the measurement period may be set arbitrarily or may be a session in which the target person does training of a specific event.

After the training of the target person ends and the measurement period ends, the exercise intensity calculation unit 14 calculates a maximum exercise intensity based on the measured heart rate (step S2). More specifically, the extraction unit 12 extracts a maximum heart rate in the measurement period from the heart rate data of the target person stored in the storage unit 11. For example, when the measurement period includes a plurality of training sessions, a maximum heart rate is extracted for each session.

Then, the exercise intensity calculation unit 14 calculates a maximum exercise intensity based on the extracted maximum heart rate using the above-described equation (1). In equation (1), "resting heart rate" and "maximum heart rate" can be actually premeasured values. The maximum exercise intensity of the target person calculated by the exercise intensity calculation unit 14 is stored in the storage unit 11.

The workload calculation unit 15 calculates a workload based on the maximum exercise intensity calculated in step S2 (step S3). More specifically, the workload calculation unit 15 calculates a workload by multiplying the length of the measurement period and the value of the maximum exercise intensity of the target person that is calculated by the exercise intensity calculation unit 14 and stored in the storage unit 11. For example, when a session is used as the unit of the measurement period, a workload is calculated for each session by multiplying the value of the maximum exercise intensity and the length of the session.

As shown in Fig. 1, the value calculated in step S3 can be used as a value equivalent to a workload obtained from the product of RPE and the time. The workload calculated by the workload calculation unit 15 may be output to the display screen by the output unit 16.

As described above, according to the first embodiment, a maximum heart rate is extracted from a heart rate measured in a period in which the target person exercises, and a workload is calculated based on a maximum exercise intensity calculated from the maximum heart rate.

The workload estimation device 1 can estimate the workload of the target person from objective data without using RPE. As result, the state of the target person is grasped based on the estimated workload, and setting of training or the like is performed properly.

[Second Embodiment]

The second embodiment will be described below. In the following description, the same reference numerals as those in the above-described first embodiment denote the same parts and a repetitive description thereof will be omitted.

In the first embodiment, a maximum heart rate is extracted from a heart rate measured in a period in which a target person trains, and a workload is estimated. In the second embodiment, a workload is calculated based on the average heart rate of the target person during exercise, and the calculated workload value is corrected.

[General Description of Second Embodiment]

First, the principle of a workload estimation device 1A according to the second embodiment will be explained with reference to Fig. 5. The abscissa of a graph shown in Fig. 5 represents a workload (predicted exercise load) obtained from the product of RPE and the time. The ordinate represents a value obtained from the product of the time and an exercise intensity calculated based on an average heart rate serving as an average of heart rates in a period in which a target person did exercise such as training. Note that an exercise intensity calculated based on the average heart rate will be particularly called an "average exercise intensity".

RPE is a value obtained using a well-known modified Borg scale. The average heart rate of a target person during exercise used to calculate an average exercise intensity on the ordinate is converted into an exercise intensity that takes a value of 0 to 10 so as to match the RPE range.

The average exercise intensity is calculated according to equation (2) (see non-patent literature 2):

$$\text{average exercise intensity} = (\text{measured average heart rate during exercise} - \text{resting heart rate})/(\text{maximum heart rate} - \text{resting heart rate}) \times 10 \quad \ldots(2)$$

where "measured average heart rate during exercise" is a value measured by a measurement unit 10 implemented by a heart rate monitor, and "resting heart rate" and "maximum heart rate" are actually premeasured values.

As shown in Fig. 5, a RPE-based workload and a value based on the average heart rate of the target person in a period in which he/she exercises are highly correlated to each other because the correlation coefficient R is close to 1. As described above, the slope of a regression line shown in Fig. 5 takes a value smaller than 1 in data in which the range of RPE and that of the average heart rate of the target person during exercise match each other.

When an average heart rate is used, a workload value is estimated to be smaller than in the first embodiment in which a maximum heart rate is used. Thus, a workload value calculated using the average heart rate of a target person is effectively corrected by multiplying it by a coefficient $\alpha$.

Since the slope of the regression line shown in Fig. 5 is 0.701, the reciprocal of the slope can be used as the correction coefficient $\alpha$ = 1/0.701. For example, a workload is calculated by multiplying the coefficient $\alpha$ and the product of the time and the average exercise intensity of the target person calculated using the above-described equation (2). This yields a corrected workload.

[Functional Blocks of Workload Estimation Device]

As shown in Fig. 6, the workload estimation device 1A according to the second embodiment is different from the workload estimation device 1 according to the first embodiment in that an estimation unit 13A further includes a correction unit 17 (Fig. 2). Functions different from those in the first embodiment will be mainly described.

Information of RPE obtained in advance from a target person is stored in a storage unit 11. Also, a workload obtained from the product of RPE and the time is stored in advance in the storage unit 11.

An extraction unit (second extraction unit) 12 extracts an average heart rate in a measurement period from the heart rate of the target person measured by the measurement unit 10 in the measurement period. Note that the extraction unit 12 can set a time of an arbitrary length as the measurement period. It is also possible to use a training session as the measurement period and extract an average heart rate for each session.

An exercise intensity calculation unit 14 calculates the average exercise intensity of the target person using the above-described equation (2).

A workload calculation unit 15 calculates a workload by multiplying the average exercise intensity of the target person calculated by the exercise intensity calculation unit 14 and the length of the measurement period serving as the training time.

The correction unit 17 corrects an average exercise intensity-based workload value calculated by the workload calculation unit 15. More specifically, the correction unit 17 obtains a relationship between the workload that is obtained from the product of RPE and the time and stored in advance in the storage unit 11, and the average exercise intensity-based workload calculated by the workload calculation unit 15.

The correction unit 17 obtains, for example, the slope of the regression line from the relationship shown in Fig. 5. The correction unit 17 uses the reciprocal of the obtained slope as the correction coefficient $\alpha$. The correction unit 17 calculates a corrected workload value by multiplying the coefficient $\alpha$ and the average exercise intensity-based workload value calculated by the workload calculation unit 15.

[Operation of Workload Estimation Device]

Next, the operation of the workload estimation device 1A having the above-described arrangement will be described with reference to the flowchart of Fig. 7.

First, a target person wears the measurement unit 10 implemented by a heart rate monitor, and starts exercise such as training.

The measurement unit 10 measures the heart rate of the target person in a period in which he/she exercises (step S20). The extraction unit 12 extracts an average heart rate in the measurement period from the measured heart rate. Note that the measurement period can be a session of training of the target person.

The exercise intensity calculation unit 14 calculates an average exercise intensity based on the average heart rate of the target person using the above-described equation (2) (step S21). The workload calculation unit 15 calculates a workload from the produce of the average exercise intensity and the length of the measurement period (step S22).

The correction unit 17 obtains a relationship between the workload that is obtained from the product of RPE and the time and stored in the storage unit 11, and the average exercise intensity-based workload calculated by the workload calculation unit 15. From the obtained relationship, the correction unit 17 obtains the coefficient $\alpha$ for correcting the workload value calculated by the workload calculation unit 15. The correction unit 17 corrects the workload value by multiplying the coefficient $\alpha$ and the average exercise intensity-based workload calculated by the workload calculation unit 15 (step S23).

As described above, according to the second embodiment, a workload is calculated based on an average heart rate serving as an average of heart rates of a target person during exercise, and a corrected value of the calculated workload is obtained. Even when the average heart rate, which is objective data, is used, the workload of the target person can be estimated.

In the above-described embodiment, the estimation unit 13A estimates a workload using the average heart rate of a target person. In the invention the estimation unit 13A uses both the average and maximum heart rates of the target person, estimate workloads based on the respective heart rates, and output them as indices for setting a load of training of the target person.

More specifically, the average heart rate is the average of heart rates in a measurement period in which a target person trained, as described above. The maximum heart rate is a heart rate when the load on the target person is heaviest in the measurement period in which he/she trained. If the difference of the maximum heart rate from the average heart rate increases, an instantaneous training load tends to increase. If the difference of the maximum heart rate from the average heart rate decreases, training tends to put a heavier load on the target person in total.

Considering this, the workload estimation device 1A outputs as indices the values of both a workload calculated based on the maximum heart rate of the target person and a workload calculated based on the average heart rate. The training load on the target person can be set more properly.

For example, when training of a heavy load in total is to be set, the exercise intensity, time, and the like of training are so adjusted as to decrease the difference between the value of a workload obtained from the product of the maximum exercise intensity and the time and the value of a workload obtained from the product of the average exercise intensity and the time.

When training of an instantaneously heavy load is to be set, the exercise intensity, time, and the like of training are so adjusted as to increase the difference between the value of a workload obtained from the product of the maximum exercise intensity and the time and the value of a workload obtained from the product of the average exercise intensity and the time.

[Third Embodiment]

The third embodiment will be described below. In the following description, the same reference numerals as those in the above-described first and second embodiments denote the same parts and a repetitive description thereof will be omitted.

In the second embodiment, a workload is calculated based on the average heart rate of a target person in a measurement period. In the third embodiment, a workload is calculated based on a measured heart rate of the target person, and compared with the value of a workload (predicted exercise load) obtained in advance from the product of pre-acquired RPE and the time.

[Functional Blocks of Workload Estimation Device]

Fig. 8 is a block diagram showing the functional arrangement of a workload estimation device 1B according to the third embodiment.

Information of RPE of a target person is stored in advance in a storage unit 11. The predicted value of a workload obtained from the product of RPE and the time is also stored in the storage unit 11.

An extraction unit 12 extracts a maximum heart rate or an average heart rate from the heart rate of the target person measured by a measurement unit 10 in a measurement period in which he/she trained. Note that the extraction unit 12 may extract a maximum heart rate or an average heart rate from the heart rate of the target person in each session.

An exercise intensity calculation unit 14 calculates a maximum exercise intensity using the above-described equation (1) based on the maximum heart rate extracted by the extraction unit 12. Note that the exercise intensity calculation unit 14 may calculate an average exercise intensity using the above-described equation (2) based on the average heart rate.

A workload calculation unit 15 calculates a workload by multiplying the length of the measurement period and the maximum exercise intensity or average exercise intensity calculated by the exercise intensity calculation unit 14. When a session is used as the measurement period, a workload is calculated for each session by multiplying the length of the session and the maximum exercise intensity or average exercise intensity.

A comparison unit 18 compares the predicted value (predicted exercise load) of a workload that is obtained from the product of RPE and the time and stored in advance in the storage unit 11, with a workload value calculated based on a measured heart rate.

More specifically, the comparison unit 18 compares the predicted value of a workload obtained from the product of RPE and the time with a workload value calculated by the workload calculation unit 15 based on a maximum heart rate. The comparison unit 18 may compare the predicted value of a workload obtained from the product of RPE and the time with a workload value calculated by the workload calculation unit 15 based on an average heart rate. Note that the workload value calculated based on the average heart rate can be a value corrected by a correction unit 17, as described in the second embodiment.

The comparison unit 18 obtains, as a comparison result, a deviation between the predicted value of the workload obtained from the product of RPE and the time, and the workload calculated by the workload calculation unit 15 based on the measured heart rate. As described above, a workload obtained from the product of the time and an exercise intensity calculated from a heart rate, and a workload obtained from the product of RPE and the time are correlated. Based on this correlation, the comparison unit 18 outputs a comparison result available to grasp the state of the target person and adjust the training load.

[Operation of Workload Estimation Device]

Next, the operation of the workload estimation device 1B according to this embodiment having the above-described arrangement will be described with reference to the flowchart of Fig. 9.

First, a target person wears the measurement unit 10 implemented by a heart rate monitor, and starts exercise such as training.

The measurement unit 10 measures the heart rate of the target person in a period in which he/she exercises (step S30). The extraction unit 12 extracts a maximum heart rate or an average heart rate in the measurement period from the measured heart rate.

Then, the exercise intensity calculation unit 14 calculates a maximum exercise intensity using the above-described equation (1) (step S31). Note that the exercise intensity calculation unit 14 may calculate an average exercise intensity. The workload calculation unit 15 calculates a workload by multiplying the calculated maximum exercise intensity or average exercise intensity and the length of the measurement period (step S32).

The comparison unit 18 compares the predicted value of a workload that is obtained from the product of RPE and the time and stored in advance in the storage unit 11, with the workload value calculated in step S32 (step S33). The comparison unit 18 obtains, as a comparison result, information representing a deviation between the predicted value of the workload obtained from the product of RPE and the time, and the workload value calculated by the workload calculation unit 15.

An output unit 16 outputs the result of comparison by the comparison unit 18 to a display screen or the like (step S34). For example, the output unit 16 may display on the display screen a numerical value representing a difference between the predicted value of the workload obtained from the product of RPE and the time, and the workload value calculated in step S32.

For example, when the predicted workload value obtained from the product of RPE and the time is larger than the workload value calculated in step S32, the target person may intend not to exert his/her power or may be injured.

When the predicted workload value obtained from the product of RPE and the time is smaller than the workload value calculated in step S32, the target person may try too hard and overwork or may be injured.

As described above, according to the third embodiment, the estimated workload of a target person is compared with the predicted value of a workload obtained from the product of RPE and the time based on the correlation between the workload estimated based on the heart rate and the workload obtained from the product of RPE and the time. For example, when an estimated workload for a given target person deviates from a correlation line, the result of comparison by the comparison unit 18 can be used to grasp the state of the target person and adjust the training load.

Information representing a deviation between the predicted value of a workload and a workload estimated from the heart rate can be used to take a measure, for example, doubt an injury of the target person. Hence, the state of the target person can be managed more properly.

[Fourth Embodiment]

The fourth embodiment will be described below. In the following description, the same reference numerals as those in the above-described first to third embodiments denote the same parts and a repetitive description thereof will be omitted. A workload estimation device 1 according to the fourth embodiment has the same arrangement as that according to the first embodiment.

In the first to third embodiments, a workload in a period in which a target person does exercise such as training, that is, in a measurement period is calculated. A training session is used as the measurement period, and a workload is calculated for each session. In the fourth embodiment, a session in which a target person does exercise such as training of a specific event is subdivided into a plurality of section times, and a workload is calculated for each section.

Fig. 10 is a graph for explaining the workload estimation device 1 according to the fourth embodiment. In Fig. 10, the abscissa represents a workload value (predicted exercise load) obtained from the product of RPE and the time. The ordinate represents a workload value obtained from the product of the time and a maximum exercise intensity calculated based on a measured heart rate.

In this embodiment, a section obtained by subdividing a session in which a target person does training of a specific event is used as the unit of the measurement period in which a measurement unit 10 measures a heart rate.

The section is, for example, a measurement period obtained by dividing one training session into every 10 minutes. The training session can be divided into every arbitrary time such as every 20 minutes or every hour depending on the event of training, its properties, and the like. This is because the heart rate can always be measured unlike RPE and can be easily handled as data in every desired time unit.

The measurement unit 10 measures the heart rate of the target person for each section.

An extraction unit 12 extracts a maximum heart rate in each section from data of the heart rate measured by the measurement unit 10.

An exercise intensity calculation unit 14 calculates a maximum exercise intensity in each section based on the maximum heart rate extracted for each section. For example, when one session is formed from a 60-min training, maximum exercise intensities in six sections obtained by dividing one session into every 10 minutes are calculated.

A workload calculation unit 15 calculates a workload for the maximum exercise intensity in each section calculated by the exercise intensity calculation unit 14. The workload calculation unit 15 calculates a workload for each section by multiplying the maximum exercise intensity in each section and the length of the section time. In the above example, six workloads in the respective 10-minute sections are calculated.

For a workload calculated based on the heart rate of a target person, a higher sampling rate can be set compared to a workload using RPE. For example, a workload is calculated for each section obtained by dividing one training session. When a target person is, for example, an athlete who plays a game in team sports, a moving amount from the start of the game can be estimated more finely in a session of one game. A workload value calculated for each section based on the heart rate of the target person can be used as a more real-time index such as an index for player change.

An actual time of a game is divided into a predetermined time unit, and the workload of a player is calculated. In training, the player can bodily sense a workload equal to or heavier than the workload in the divided time unit and can adapt to the game. In this manner, a workload calculated in each divided time unit can be used for more detailed setting of a training load on a target person such as an athlete.

In this embodiment, a workload calculated based on a maximum exercise intensity is used as shown in Fig. 10. Alternatively, a workload based on an average exercise intensity calculated from an average heart rate may be used.

In the above-described embodiment, a workload is calculated for each section obtained by dividing, by a fixed interval, a period in which a heart rate is measured. However, the above-mentioned section is not limited to a fixed time interval. For example, it is also possible to pay attention to categories constituting training of one event in a session of this training, and divide one session into a plurality of sections. For example, in a training session of an event "1500m freestyle swim", one session can be divided into a plurality of sections based on swimming styles such as crawl, breaststroke, butterfly, and backstroke. In this case, the lengths of the sections may be different from each other.

[Fifth Embodiment]

The fifth embodiment will be described below. In the following description, the same reference numerals as those in the above-described first to fourth embodiments denote the same parts and a repetitive description thereof will be omitted.

In the first to third embodiments, a workload in a measurement period in which a target person does exercise such as training is calculated. Further, in the first to third embodiments, the measurement period includes a training session and a workload is calculated for each session. In the fourth embodiment, a workload is calculated for each section time obtained by subdividing the training session. To the contrary, in the fifth embodiment, the sum of workloads calculated for respective sessions or sections is calculated in a predetermined time unit (each predetermined period).

As shown in Fig. 11, a workload estimation device 1C according to this embodiment is different from the first to fourth embodiments in that an estimation unit 13C includes a sum calculation unit 19. An arrangement different from those in the first to fourth embodiments will be mainly described.

In this embodiment, an extraction unit 12 extracts a maximum heart rate or an average heart rate in each measurement period from data of the heart rate of a target person measured by a measurement unit 10 in each measurement period such as each training session. The extraction unit 12 may extract a maximum heart rate or an average heart rate in each section obtained by dividing one session in a smaller time unit.

An exercise intensity calculation unit 14 calculates an exercise intensity for each session or each section extracted by the extraction unit 12. The exercise intensity calculation unit 14 can calculate a maximum exercise intensity or an average exercise intensity for each session or each section.

A workload calculation unit 15 calculates a workload of the exercise intensity calculated for each session or each section.

The sum calculation unit 19 calculates the sum of workloads in a predetermined time unit. For example, a case will be considered in which a workload is calculated for each session and the sum of workloads is calculated for a day serving as the predetermined time unit. In this case, the sum calculation unit 19 can calculate the sum of workloads a day according to equation (3):

$$\text{one-day workload} = \Sigma(\text{exercise intensity} \times \text{session time}) \quad ...(3)$$

When calculating a workload for each section obtained by subdividing a training session, the sum of workloads a day is calculated by multiplying the exercise intensity in each section and the section time.

The sum of workloads calculated by the sum calculation unit 19 in a predetermined time unit such as a day is stored in a storage unit 11.

Next, the operation of the workload estimation device 1C according to this embodiment will be described with reference to the flowchart of Fig. 12. A case in which the workload estimation device 1C outputs the sum of workloads a day will be exemplified.

First, a target person wears a measurement unit 10 implemented by a heart rate monitor, and starts exercise such as training.

The measurement unit 10 measures the heart rate of the target person in a period in which he/she exercises (step S50). For example, the target person wears the measurement unit 10 while he/she exercises. The measurement unit 10 can add information representing the measurement date and time to the measured heart rate data. Then, the extraction unit 12 extracts, from the measured heart rate, a maximum heart rate or an average heart rate for each session or each section serving as a time unit obtained by subdividing the session.

For example, a case will be considered in which a maximum heart rate or an average heart rate is extracted from a heart rate in each session. For example, when there are four 60-min training sessions a day, four maximum heart rates or average heart rates are extracted from measured heart rates. Note that information about the number of sessions a day or the length of the session time is stored in advance in the storage unit 11.

After that, the exercise intensity calculation unit 14 calculates a maximum exercise intensity or an average exercise intensity using the above-mentioned equation (1) or (2) (step S51). Note that the exercise intensity calculation unit 14 may calculate an average exercise intensity. The workload calculation unit 15 calculates a workload for each session or each section by multiplying the calculated maximum exercise intensity or average exercise intensity and the length of the session or the length of the section time (step S52).

The sum calculation unit 19 calculates using the above-described equation (3) the sum of workloads a day that have been calculated for respective sessions or respective sections in step S52 (step S53). Then, an output unit 16 may display on a display screen the sum of workloads a day that has been calculated in step S53.

As described above, according to the fifth embodiment, the sum of workloads in a predetermined time unit such as a day is calculated. Data of a workload corresponding to a time unit commonly used in planning and management of training or the like can be output. As a result, the workload calculated based on objective data can be provided as data more easily available for planning and scheduling of training.

For example, when planning training, the training amount can be managed by setting a threshold for a workload a day based on the sum of workloads a day obtained in this embodiment. More specifically, when a workload a day exceeds the threshold, the output unit 16 may output an alert or the like on the display screen based on the set threshold together with the calculated workload.

Note that the sum calculation unit 19 according to the above-described fifth embodiment can also be combined with the arrangement described in each of the second to fourth embodiments.

[Sixth Embodiment]

The sixth embodiment will be described below. In the following description, the same reference numerals as those in the above-described first to fifth embodiments denote the same parts and a repetitive description thereof will be omitted.

In the fifth embodiment, the sum of workloads in a predetermined time unit such as a day is calculated. In the sixth embodiment, the average (to be referred to as an acute workload (acute load)) of workloads in a preset short period (first period) and the average (to be referred to as a chronic workload (chronic load)) of workloads in a longer period (second period) are calculated based on the sum of workloads in a predetermined time unit. Further, in the sixth embodiment, the ratio (to be referred to as an "A/C ratio" hereinafter) between the acute workload and the chronic workload is calculated.

The acute workload is a value representing the average of workloads in a period, typically a week, that is relatively short for training of an athlete. The acute workload represents the fatigue of a target person such as an athlete who trains (see non-patent literature 1). The chronic workload is a value representing the average of workloads in a period, typically four weeks, that is longer than the period of the acute workload. The chronic workload represents a positive effect such as a healthy and appropriate state of a target person who trains (see non-patent literature 1).

The A/C ratio (Acute : Chronic workload ratio) serving as the ratio between the acute workload and the chronic workload is used as an index for predicting the occurrence of an injury of a target person who trains, such as an athlete. It is known that the injury occurrence frequency can be reduced by managing the training amount at an A/C ratio of 0.8 to 1.3 (see non-patent literature 1).

The period in which the acute workload is calculated needs to be shorter than the period in which the chronic workload is calculated. These periods are properly changed in accordance with the training schedule of the target person or the like.

As shown in Fig. 13, a workload estimation device 1D according to this embodiment is different from the fifth embodiment in that an estimation unit 13D includes an average calculation unit 20. An arrangement different from that in the fifth embodiment will be mainly described.

A sum calculation unit 19 calculates the sum of workloads in a predetermined time unit. For example, the sum calculation unit 19 can calculate the sum of workloads a day according to the above-mentioned equation (3). The sum of workloads a day calculated by the sum calculation unit 19 is stored in a storage unit 11.

The average calculation unit 20 calculates an acute workload and a chronic workload based on, for example, the sum of workloads a day calculated by the sum calculation unit 19. The average calculation unit 20 further calculates an A/C ratio. As for the value of the chronic workload, a value prepared in advance as an initial value may be used.

More specifically, the average calculation unit 20 calculates the average of workloads a week based on workloads in a preset number of days, for example, seven days that are calculated by the sum calculation unit 19 and stored in the storage unit 11. The average calculation unit 20 outputs the average as an acute workload. The calculated acute workload is stored in the storage unit 11.

The average calculation unit 20 calculates the average of workloads in a preset period, for example, four weeks based on a workload a day or the average of workloads a week stored in the storage unit 11, and outputs the average as a chronic workload. The calculated chronic workload is also stored in the storage unit 11.

The average calculation unit 20 calculates an A/C ratio based on the calculated acute workload and chronic workload. To calculate the acute workload with respect to the chronic workload, the average calculation unit 20 may calculate an A/C ratio upon, for example, calculating a chronic workload serving as the average of workloads in four weeks. The average calculation unit 20 may calculate an A/C ratio using an initial value prepared in advance as the chronic workload. The A/C ratio can be calculated based on, for example, a moving average model or an exponentially weighted moving average model.

Next, the operation of the workload estimation device 1D according to this embodiment will be described with reference to the flowchart of Fig. 14. A case will be exemplified in which the workload estimation device 1D outputs the average of workloads a week as an acute workload and calculates the average of workloads in four weeks as a chronic workload.

First, a target person wears a measurement unit 10 implemented by a heart rate monitor, and starts training or the like.

The measurement unit 10 measures the heart rate of the target person in a period in which he/she exercises (step S60). For example, the target person wears the measurement unit 10 while he/she trains. The measurement unit 10 can add information about the measurement date and time to the measured heart rate data. Then, the extraction unit 12 extracts, from the measured heart rate, a maximum heart rate or an average heart rate for each session or each section serving as a time unit obtained by subdividing the session.

An exercise intensity calculation unit 14 calculates a maximum exercise intensity or an average exercise intensity using the above-mentioned equation (1) or (2) (step S61). Note that the exercise intensity calculation unit 14 may calculate an average exercise intensity. A workload calculation unit 15 calculates a workload for each session or each section by multiplying the calculated maximum exercise intensity or average exercise intensity and the length of the session time or the length of the section time (step S62).

The sum calculation unit 19 calculates using the above-described equation (3) the sum of workloads a day that have been calculated for respective sessions or respective sections in step S62 (step S63). The calculated sum of workloads a day is stored in the storage unit 11.

After workloads in seven days are stored in the storage unit 11, the average calculation unit 20 calculates an acute workload serving as the average of workloads a week and stores it in the storage unit 11 (step S64). After workloads in four weeks are stored in the storage unit 11, the average calculation unit 20 calculates a chronic workload serving as the average of workloads in four weeks and stores it in the storage unit 11 (step S65).

The average calculation unit 20 calculates an A/C ratio based on the acute workload and chronic workload calculated respectively in step S64 and step S65 (step S66). More specifically, the average calculation unit 20 calculates an A/C ratio by dividing the acute workload by the chronic workload. The calculated A/C ratio is stored in the storage unit 11.

In this manner, the sum calculation unit 19 calculates the sum of workloads a day based on data of the heart rate of a target person. The average calculation unit 20 calculates an acute workload, a chronic workload, and an A/C ratio based on the workloads of respective days stored in the storage unit 11, and sequentially updates these values.

After that, an output unit 16 can display the A/C ratio calculated in step S66 on a display screen together with information representing transition of the acute workload and chronic workload.

As described above, according to the sixth embodiment, the workload estimation device 1D calculates an acute workload and a chronic workload based on the sum of workloads a day. Based on the calculated acute workload and chronic workload, the workload estimation device 1D calculates an A/C ratio serving as an index for predicting the occurrence of an injury of an athlete or the like who trains.

The training load on the athlete can be more properly managed based on the acute workload, chronic workload, and A/C ratio output from the workload estimation device 1D, and the occurrence frequency of injuries can be reduced.

Note that the above-described sixth embodiment can also be adopted in combination with each of the second to fourth embodiments.

Although the embodiments of the exercise load estimation method and exercise load estimation device have been described above, the invention is not limited to these embodiments. It will be understood by a person of ordinary skill in the art that various changes in form and details may be made therein without departing from the scope of the present invention as defined by the claims.

Explanation of the Reference Numerals and Signs

1, 1A, 1B...workload estimation device, 10...measurement unit, 11...storage unit, 12... extraction unit, 13, 13A...estimation unit, 14...exercise intensity calculation unit, 15...workload calculation unit, 16...output unit, 17...correction unit, 18...comparison unit, 101...bus, 102...arithmetic device, 103...CPU, 104...main storage device, 105...communication control device, 106...sensor, 107...external storage device, 107a...data storage, 107b...program storage, 108...timer, 109...display device, NW... communication network

## Claims

1. A computer-implemented exercise load estimation method comprising:

   a measurement step of measuring a heart rate of a target person who exercises; and
   an estimation step of estimating an exercise load on the target person based on the measured heart rate and a length of a measurement period in which the heart rate was measured,
   the estimation step including:

      an exercise intensity calculation step of calculating an exercise intensity of the target person from the heart rate measured in the measurement step; and
      an exercise load calculation step of calculating the exercise load based on the calculated exercise intensity and the length of the measurement period,
      wherein the measurement step includes a first extraction step of extracting a maximum heart rate of the target person in the measurement period from the measured heart rate, and
      in the exercise intensity calculation step, the exercise intensity is calculated from the maximum heart rate,
      **characterized in that**
      the measurement step includes a second extraction step of extracting an average heart rate of the target person in the measurement period from the measured heart rate,
      in the exercise intensity calculation step, the exercise intensity is calculated from the average heart rate,
      the estimation step further includes a correction step of correcting a value of the exercise load calculated in the exercise load calculation step, and
      in the correction step, the value of the exercise load is corrected based on a relationship between the exercise load calculated in the exercise load calculation step, and a predicted exercise load, which is stored in advance in a storage unit, based on a rating of perceived exertion representing an exercise intensity perceived by the target person.

2. The exercise load estimation method according to claim 1, further comprising:

   a comparison step of comparing the exercise load estimated in the estimation step with a predicted exercise load based on a rating of perceived exertion representing an exercise intensity perceived by the target person; and
   an output step of outputting a comparison result in the comparison step.

3. An exercise load estimation device (1, 1A, 1B) comprising:

a measurement unit (10) configured to measure a heart rate of a target person who exercises; and
an estimation unit (13, 13A, 13C, 13D) configured to estimate an exercise load on the target person based on the measured heart rate and a length of a measurement period,
the estimation unit (13, 13A, 13C, 13D) including:

an exercise intensity calculation unit (14) configured to calculate an exercise intensity of the target person from the heart rate measured by the measurement unit; and
an exercise load calculation unit (15) configured to calculate the exercise load based on the calculated exercise intensity and the length of the measurement period,
wherein the measurement unit (10) includes a first extraction unit configured to extract a maximum heart rate of the target person from the measured heart rate, and
the exercise intensity calculation unit (14) calculates the exercise intensity from the maximum heart rate,
**characterized in that**
the measurement unit (10) includes a second extraction unit (12) configured to extract an average heart rate of the target person in the measurement period from the measured heart rate,
the exercise intensity calculation unit (14) calculates the exercise intensity from the average heart rate,
the estimation unit (13, 13A, 13C, 13D) further includes a correction unit (17) configured to correct a value of the exercise load calculated by the exercise load calculation unit,
the correction unit (17) is configured to correct the value of the exercise load based on a relationship between the exercise load calculated by the exercise load calculation unit (15), and a predicted exercise load, which is stored in advance in a storage unit (11), based on a rating of perceived exertion representing an exercise intensity perceived by the target person

4. The exercise load estimation device according to claim 3, further comprising a sum calculation unit configured to calculate a sum of the exercise loads calculated by the exercise load calculation unit (15) in each predetermined period.

5. The exercise load estimation device according to claim 4, further comprising an average calculation unit configured to calculate, based on the sum of the exercise loads calculated by the sum calculation unit (19) in each predetermined period, an acute exercise load serving as an average of the exercise loads in a first period having at least a length of the predetermined period, and a chronic exercise load serving as an average of the exercise loads in a second period longer than the first period, and calculate an A/C ratio representing a ratio of the acute exercise load to the chronic exercise load.

6. A computer-readable recording medium storing a program executable in an exercise load estimation device according to claim 3 that estimates an exercise load on a target person who exercises, wherein the program includes:

a measurement step of measuring a heart rate of the target person who exercises; and
an estimation step of estimating the exercise load on the target person based on the measured heart rate and a length of a measurement period in which the heart rate was measured, and
the estimation step includes:

an exercise intensity calculation step of calculating an exercise intensity of the target person from the heart rate measured in the measurement step; and
an exercise load calculation step of calculating the exercise load based on the calculated exercise intensity and the length of the measurement period,
wherein
the measurement step includes a first extraction step of extracting a maximum heart rate of the target person in the measurement period from the measured heart rate, and
in the exercise intensity calculation step, the exercise intensity is calculated from the maximum heart rate
**characterized in that**
the measurement step includes a second extraction step of extracting an average heart rate of the target person in the measurement period from the measured heart rate,
in the exercise intensity calculation step, the exercise intensity is calculated from the average heart rate,
the estimation step further includes a correction step of correcting a value of the exercise load calculated in the exercise load calculation step, and

in the correction step, the value of the exercise load is corrected based on a relationship between the exercise load calculated in the exercise load calculation step, and a predicted exercise load, which is stored in advance in a storage unit, based on a rating of perceived exertion representing an exercise intensity perceived by the target person.

**Patentansprüche**

1. Computer-implementiertes Verfahren zur Schätzung der Trainingsbelastung, umfassend:

    einen Messschritt zum Messen einer Herzfrequenz einer Zielperson, die trainiert; und
    einen Schätzschritt zum Schätzen einer Trainingsbelastung für die Zielperson auf Grundlage der gemessenen Herzfrequenz und einer Länge einer Messperiode, in der die Herzfrequenz gemessen wurde,
    wobei der Schätzschritt Folgendes umfasst:

        einen Berechnungsschritt für die Trainingsintensität zum Berechnen einer Trainingsintensität der Zielperson aus der im Messschritt gemessenen Herzfrequenz; und
        einen Berechnungsschritt für die Trainingsbelastung zum Berechnen der Trainingsbelastung auf Grundlage der berechneten Trainingsintensität und der Länge der Messperiode,
        wobei der Messschritt einen ersten Extrahierungsschritt des Extrahierens einer maximalen Herzfrequenz der Zielperson in der Messperiode aus der gemessenen Herzfrequenz umfasst, und
        im Berechnungsschritt der Trainingsintensität die Trainingsintensität aus der maximalen Herzfrequenz berechnet wird,
        **dadurch gekennzeichnet, dass**
        der Messschritt einen zweiten Extrahierungsschritt des Extrahierens einer in der Messperiode durchschnittlichen Herzfrequenz der Zielperson aus der gemessenen Herzfrequenz umfasst,
        im Berechnungsschritt der Trainingsintensität die Trainingsintensität aus der durchschnittlichen Herzfrequenz berechnet wird,
        der Schätzschritt ferner einen Korrekturschritt des Korrigierens eines Wertes der Trainingsbelastung, die im Berechnungsschritt der Trainingsbelastung berechnet wurde, umfasst, und
        im Korrekturschritt der Wert der Trainingsbelastung auf der Grundlage einer Beziehung zwischen der im Berechnungsschritt für die Trainingsbelastung berechneten Trainingsbelastung und einer vorhergesagten Trainingsbelastung, die im Voraus in einer Speichereinheit gespeichert wird, auf der Grundlage einer Bewertung der wahrgenommenen Anstrengung, die eine von der Zielperson wahrgenommene Trainingsintensität darstellt, korrigiert wird.

2. Verfahren zur Abschätzung der Trainingsbelastung
nach Anspruch 1, ferner umfassend:

    einen Vergleich-Schritt zum Vergleichen der im Schätzschritt geschätzten Trainingsbelastung mit einer vorhergesagten Trainingsbelastung auf Grundlage einer Bewertung der wahrgenommenen Anstrengung, die eine von der Zielperson wahrgenommene Trainingsintensität darstellt; und
    einen Ausgabeschritt zum Ausgeben eines Vergleichsergebnisses im Vergleichsschritt.

3. Vorrichtung zur Schätzung der Trainingsbelastung (1, 1A, 1B), umfassend:

    eine Messeinheit (10), die so konfiguriert ist, dass sie eine Herzfrequenz einer Zielperson, die trainiert, misst; und
    eine Schätzeinheit (13, 13A, 13C, 13D), die so konfiguriert ist, dass sie eine Trainingsbelastung für die Zielperson auf Grundlage der gemessenen Herzfrequenz und einer Länge einer Messperiode schätzt,
    wobei die Schätzeinheit (13, 13A, 13C, 13D) umfasst:

        eine Berechnungseinheit (14) für die Trainingsintensität, die so konfiguriert ist, dass sie eine Trainingsintensität der Zielperson aus der von der Messeinheit gemessenen Herzfrequenz berechnet; und
        eine Berechnungseinheit (15) für die Trainingsbelastung, die so konfiguriert ist, dass sie die Trainingsbelastung auf Grundlage der berechneten Trainingsintensität und der Länge der Messperiode berechnet,
        wobei die Messeinheit (10) eine erste Extraktionseinheit enthält, die konfiguriert ist, um eine maximale Herzfrequenz der Zielperson aus der gemessenen Herzfrequenz zu extrahieren, und
        die Berechnungseinheit (14) für die Trainingsintensität die Trainingsintensität aus der maximalen Herzfrequenz berechnet,
**dadurch gekennzeichnet, dass**
die Messeinheit (10) eine zweite Extraktionseinheit (12) enthält, die so konfiguriert ist, dass sie eine durchschnittliche Herzfrequenz der Zielperson in der Messperiode aus der gemessenen Herzfrequenz extrahiert,
die Berechnungseinheit (14) für die Trainingsintensität die Trainingsintensität aus der durchschnittlichen Herzfrequenz berechnet,
die Schätzeinheit (13, 13A, 13C, 13D) ferner eine Korrektureinheit (17) umfasst, die so konfiguriert ist, dass sie einen Wert der von der Berechnungseinheit für die Trainingsbelastung berechneten Trainingsbelastung korrigiert,
die Korrektureinheit (17) so konfiguriert ist, dass sie den Wert der Trainingsbelastung auf Grundlage einer Beziehung zwischen der von der Berechnungseinheit (15) für die Trainingsbelastung berechneten Trainingsbelastung und einer vorhergesagten Trainingsbelastung, die im Voraus in einer Speichereinheit (11) gespeichert ist, auf Grundlage einer Bewertung der wahrgenommenen Anstrengung korrigiert, die eine von der Zielperson wahrgenommene Trainingsintensität darstellt.

4. Vorrichtung zur Schätzung der Trainingsbelastung
nach Anspruch 3 ferner umfassend eine Summen-Berechnungseinheit, die konfiguriert ist, um eine Summe der Trainingsbelastungen zu berechnen, die von der Trainingsbelastungs-Berechnungseinheit (15) in jeder vorbestimmten Periode berechnet wurden.

5. Vorrichtung zur Schätzung der Trainingsbelastung
nach Anspruch 4, ferner umfassend eine Durchschnittsberechnungseinheit, die so konfiguriert ist, dass sie auf der Grundlage der Summe der von der Summen-Berechnungseinheit (19) in jeder vorbestimmten Periode berechneten Trainingsbelastungen eine akute Trainingsbelastung, die als Durchschnitt der Trainingsbelastungen in einer ersten Periode mit mindestens einer Länge der vorbestimmten Periode dient, und eine chronische Trainingsbelastung, die als Durchschnitt der Trainingsbelastungen in einer zweiten Periode, die länger als die erste Periode ist, dient, berechnet und ein A/C-Verhältnis berechnet, das ein Verhältnis der akuten Trainingsbelastung zu der chronischen Trainingsbelastung darstellt.

6. Ein computerlesbares Speichermedium
auf dem ein Programm gespeichert ist, das in einer Vorrichtung zur Schätzung der Trainingsbelastung nach Anspruch 3 ausführbar ist, die eine Trainingsbelastung für eine Zielperson, die trainiert, schätzt, wobei das Programm umfasst:

einen Messschritt zum Messen einer Herzfrequenz der Zielperson, die trainiert; und
einen Schätzschritt zum Schätzen der Trainingsbelastung der Zielperson auf der Grundlage der gemessenen Herzfrequenz und einer Länge einer Messperiode, in der die Herzfrequenz gemessen wurde, und
der Schätzschritt umfasst:

einen Berechnungsschritt für die Trainingsintensität zum Berechnen einer Trainingsintensität der Zielperson aus der im Messschritt gemessenen Herzfrequenz; und
einen Berechnungsschritt für die Trainingsbelastung zum Berechnen der Trainingsbelastung auf Grundlage der berechneten Trainingsintensität und der Länge der Messperiode,
wobei
der Messschritt einen ersten Extrahierungsschritt des Extrahierens einer maximalen Herzfrequenz der Zielperson in der Messperiode aus der gemessenen Herzfrequenz umfasst, und
im Berechnungsschritt der Trainingsintensität die Trainingsintensität aus der maximalen Herzfrequenz berechnet wird
**dadurch gekennzeichnet, dass**
der Messschritt einen zweiten Extrahierungsschritt des Extrahierens einer durchschnittlichen Herzfrequenz der Zielperson in der Messperiode aus der gemessenen Herzfrequenz umfasst,
im Berechnungsschritt der Trainingsintensität die Trainingsintensität aus der durchschnittlichen Herzfrequenz berechnet wird,
der Schätzschritt ferner einen Korrekturschritt des Korrigierens eines Wertes der Trainingsbelastung, die in dem Berechnungsschritt der Trainingsbelastung berechnet wurde, umfasst, und
im Korrekturschritt der Wert der Trainingsbelastung auf der Grundlage einer Beziehung zwischen der im Berechnungsschritt für die Trainingsbelastung berechneten Trainingsbelastung und einer vorhergesagten Trainingsbelastung, die im Voraus in einer Speichereinheit gespeichert wird, auf Grundlage einer Bewertung der wahrgenommenen Anstrengung, die eine von der Zielperson wahrgenommene Trainingsintensität darstellt, korrigiert wird.

## Revendications

1. Procédé d'estimation de charge d'exercice mis en œuvre par ordinateur comprenant :

une étape de mesure consistant à mesurer une fréquence cardiaque d'une personne cible qui fait de l'exercice ; et
une étape d'estimation consistant à estimer une charge d'exercice sur la personne cible sur la base de la fréquence cardiaque mesurée et d'une longueur d'une période de mesure pendant laquelle la fréquence cardiaque a été mesurée,
l'étape d'estimation comportant :

une étape de calcul d'intensité d'exercice consistant à calculer une intensité d'exercice de la personne cible à partir de la fréquence cardiaque mesurée à l'étape de mesure ; et
une étape de calcul de charge d'exercice consistant à calculer la charge d'exercice sur la base de l'intensité d'exercice calculée et de la longueur de la période de mesure,
dans lequel l'étape de mesure comporte une première étape d'extraction consistant à extraire une fréquence cardiaque maximale de la personne cible dans la période de mesure à partir de la fréquence cardiaque mesurée, et
à l'étape de calcul d'intensité d'exercice, l'intensité d'exercice est calculée à partir de la fréquence cardiaque maximale,
**caractérisé en ce que**
l'étape de mesure comporte une deuxième étape d'extraction consistant à extraire une fréquence cardiaque moyenne de la personne cible dans la période de mesure à partir de la fréquence cardiaque mesurée,
à l'étape de calcul d'intensité d'exercice, l'intensité d'exercice est calculée à partir de la fréquence cardiaque moyenne,
l'étape d'estimation comporte en outre une étape de correction consistant à corriger une valeur de la charge d'exercice calculée à l'étape de calcul de charge d'exercice, et
à l'étape de correction, la valeur de la charge d'exercice est corrigée sur la base d'une relation entre la charge d'exercice calculée à l'étape de calcul de charge d'exercice, et une charge d'exercice prédite, qui est stockée au préalable dans une unité de stockage, sur la base d'un régime d'effort perçu représentant une intensité d'exercice perçue par la personne cible.

2. Procédé d'estimation de charge d'exercice selon la revendication 1, comprenant en outre :

une étape de comparaison consistant à comparer la charge d'exercice estimée à l'étape d'estimation à une charge d'exercice prédite sur la base d'un régime d'effort perçu représentant une intensité d'exercice perçue par la personne cible ; et
une étape de sortie consistant à délivrer un résultat de comparaison à l'étape de comparaison.

3. Dispositif d'estimation de charge d'exercice (1, 1A, 1B) comprenant :

une unité de mesure (10) configurée pour mesurer une fréquence cardiaque d'une personne qui fait de l'exercice ; et
une unité d'estimation (13, 13A, 13C, 13D) configurée pour estimer une charge d'exercice sur la personne cible sur la base de la fréquence cardiaque mesurée et d'une longueur d'une période de mesure,
l'unité d'estimation (13, 13A, 13C, 13D) comportant :

une unité de calcul d'intensité d'exercice (14) configurée pour calculer une intensité d'exercice de la personne cible à partir de la fréquence cardiaque mesurée par l'unité de mesure ; et
une unité de calcul de charge d'exercice (15) configurée pour calculer la charge d'exercice sur la base de l'intensité d'exercice calculée et de la longueur de la période de mesure,
dans lequel l'unité de mesure (10) comporte une première unité d'extraction configurée pour extraire une fréquence cardiaque maximale de la personne cible à partir de la fréquence cardiaque mesurée, et
l'unité de calcul d'intensité d'exercice (14) calcule l'intensité d'exercice à partir de la fréquence cardiaque maximale,
**caractérisé en ce que**

l'unité de mesure (10) comporte une deuxième unité d'extraction consistant (12) configurée pour extraire une fréquence cardiaque moyenne de la personne cible dans la période de mesure à partir de la fréquence cardiaque mesurée,
l'unité de calcul d'intensité d'exercice (14) calcule l'intensité d'exercice à partir de la fréquence cardiaque moyenne,
l'unité d'estimation (13, 13A, 13C, 13D) comporte en outre une unité de correction (17) configurée pour corriger une valeur de la charge d'exercice calculée par l'unité de calcul de charge d'exercice,
l'unité de correction (17) est configurée pour corriger la valeur de la charge d'exercice sur la base d'une relation entre la charge d'exercice calculée par l'unité de calcul de charge d'exercice (15), et une charge d'exercice prédite, qui est stockée au préalable dans une unité de stockage (11), sur la base d'un régime d'effort perçu représentant une intensité d'exercice perçue par la personne cible.

**4.** Dispositif d'estimation de charge d'exercice selon la revendication 3, comprenant en outre une unité de calcul de somme configurée pour calculer une somme des charges d'exercice calculées par l'unité de calcul de charge d'exercice (15) dans chaque période prédéterminée.

**5.** Dispositif d'estimation de charge d'exercice selon la revendication 4, comprenant en outre une unité de calcul de moyenne configurée pour calculer, sur la base de la somme des charges d'exercices calculée par l'unité de calcul de somme (19) dans chaque période prédéterminée, une charge d'exercice aigu servant de moyenne des charges d'exercice dans une première période présentant au moins une longueur de la période prédéterminée, et une charge d'exercice chronique servant de moyenne des charges d'exercice dans une deuxième période plus longue que la première période, et calculer un rapport A/C représentant un rapport de la charge d'exercice aigu sur la charge d'exercice chronique.

**6.** Support d'enregistrement lisible par ordinateur stockant un programme exécutable dans un dispositif d'estimation de charge d'exercice selon la revendication 3 qui estime une charge d'exercice sur une personne cible qui fait de l'exercice, dans lequel le programme comporte :

une étape de mesure consistant à mesurer une fréquence cardiaque d'une personne cible qui fait de l'exercice ; et
une étape d'estimation consistant à estimer une charge d'exercice sur la personne cible sur la base de la fréquence cardiaque mesurée et d'une longueur d'une période de mesure pendant laquelle la fréquence cardiaque a été mesurée, et
l'étape d'estimation comporte :

une étape de calcul d'intensité d'exercice consistant à calculer une intensité d'exercice de la personne cible à partir de la fréquence cardiaque mesurée à l'étape de mesure ; et
une étape de calcul de charge d'exercice consistant à calculer la charge d'exercice sur la base de l'intensité d'exercice calculée et de la longueur de la période de mesure,
dans lequel
l'étape de mesure comporte une première étape d'extraction consistant à extraire une fréquence cardiaque maximale de la personne cible dans la période de mesure à partir de la fréquence cardiaque mesurée, et à l'étape de calcul d'intensité d'exercice, l'intensité d'exercice est calculée à partir de la fréquence cardiaque maximale,
**caractérisé en ce que**
l'étape de mesure comporte une deuxième étape d'extraction consistant à extraire une fréquence cardiaque moyenne de la personne cible dans la période de mesure à partir de la fréquence cardiaque mesurée,
à l'étape de calcul d'intensité d'exercice, l'intensité d'exercice est calculée à partir de la fréquence cardiaque moyenne,
l'étape d'estimation comporte en outre une étape de correction consistant à corriger une valeur de la charge d'exercice calculée à l'étape de calcul de charge d'exercice, et
à l'étape de correction, la valeur de la charge d'exercice est corrigée sur la base d'une relation entre la charge d'exercice calculée à l'étape de calcul de charge d'exercice, et une charge d'exercice prédite, qui est stockée au préalable dans une unité de stockage, sur la base d'un régime d'effort perçu représentant une intensité d'exercice perçue par la personne cible.

# FIG.1

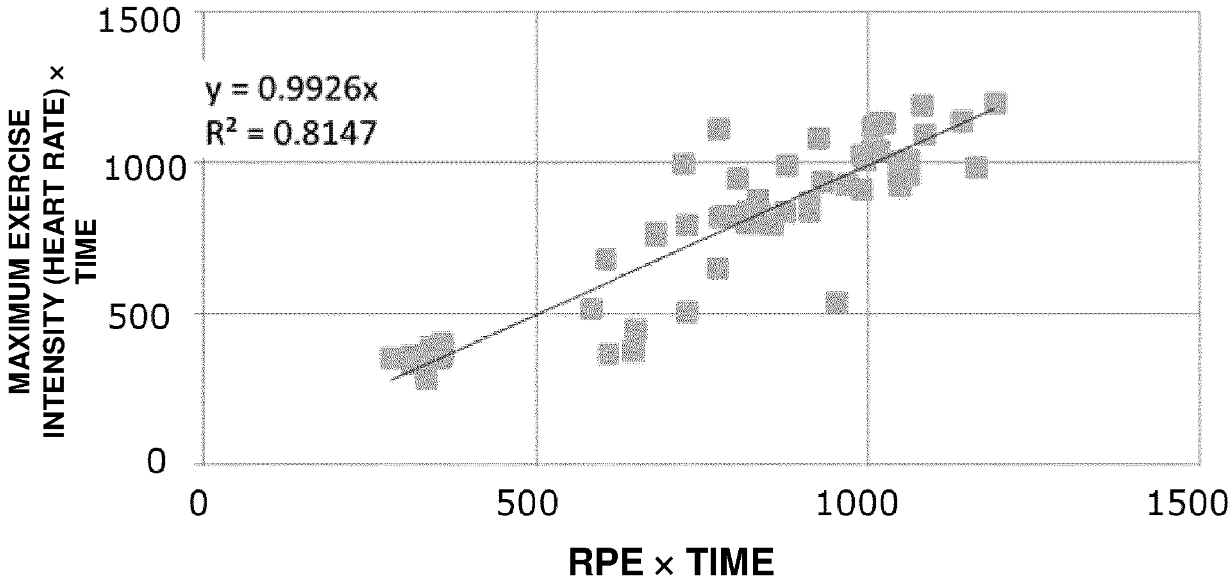
1500
1000
500
0
y = 0.9926x
R² = 0.8147
MAXIMUM EXERCISE INTENSITY (HEART RATE) × TIME
0
500
1000
1500
RPE × TIME

# FIG.2

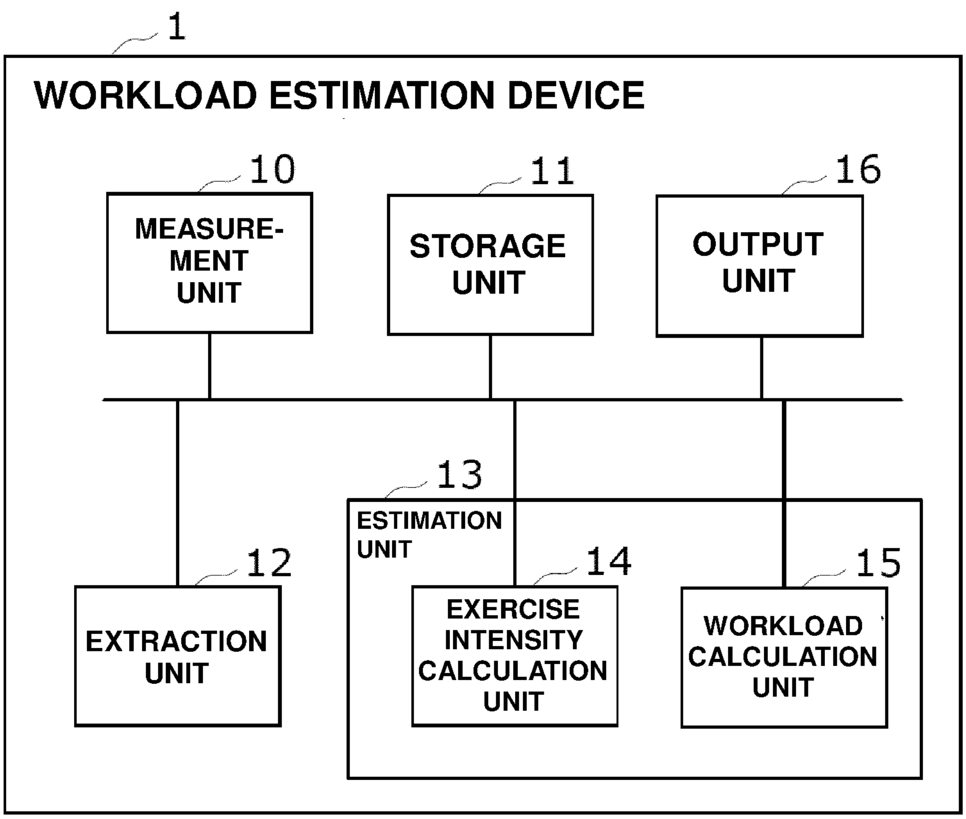
1
WORKLOAD ESTIMATION DEVICE
10
MEASURE-MENT UNIT
11
STORAGE UNIT
16
OUTPUT UNIT
13
ESTIMATION UNIT
12
EXTRACTION UNIT
14
EXERCISE INTENSITY CALCULATION UNIT
15
WORKLOAD CALCULATION UNIT

FIG.3

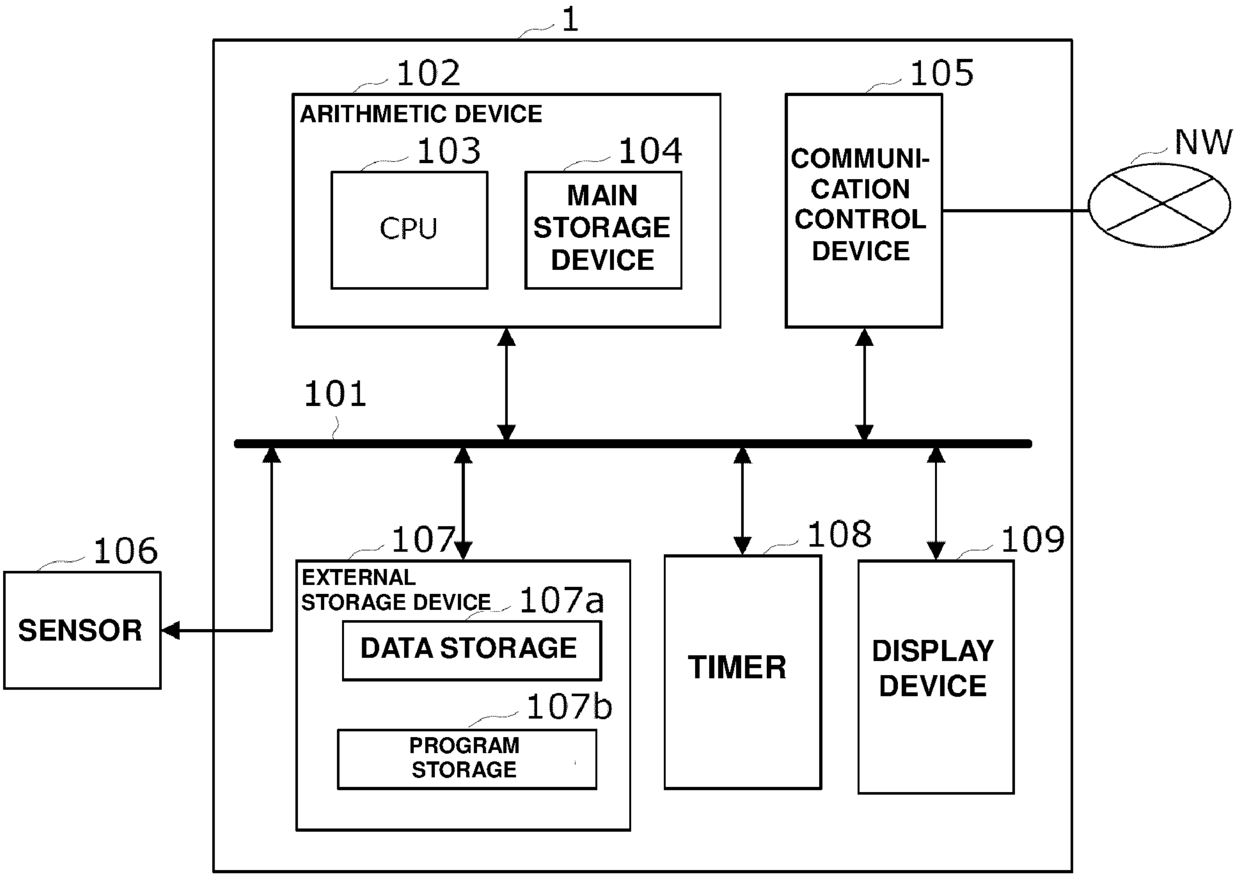
1
102
ARITHMETIC DEVICE
103
CPU
104
MAIN STORAGE DEVICE
105
COMMUNI-CATION CONTROL DEVICE
NW
101
106
SENSOR
107
EXTERNAL STORAGE DEVICE
107a
DATA STORAGE
107b
PROGRAM STORAGE
108
TIMER
109
DISPLAY DEVICE

FIG.4

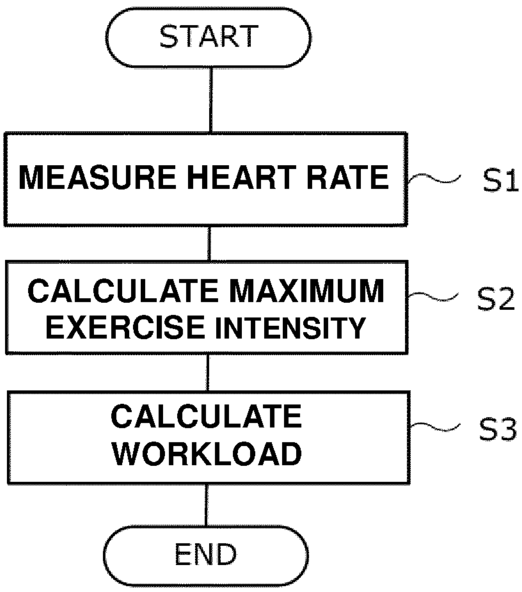
START
MEASURE HEART RATE
S1
CALCULATE MAXIMUM EXERCISE INTENSITY
S2
CALCULATE WORKLOAD
S3
END

# FIG.5

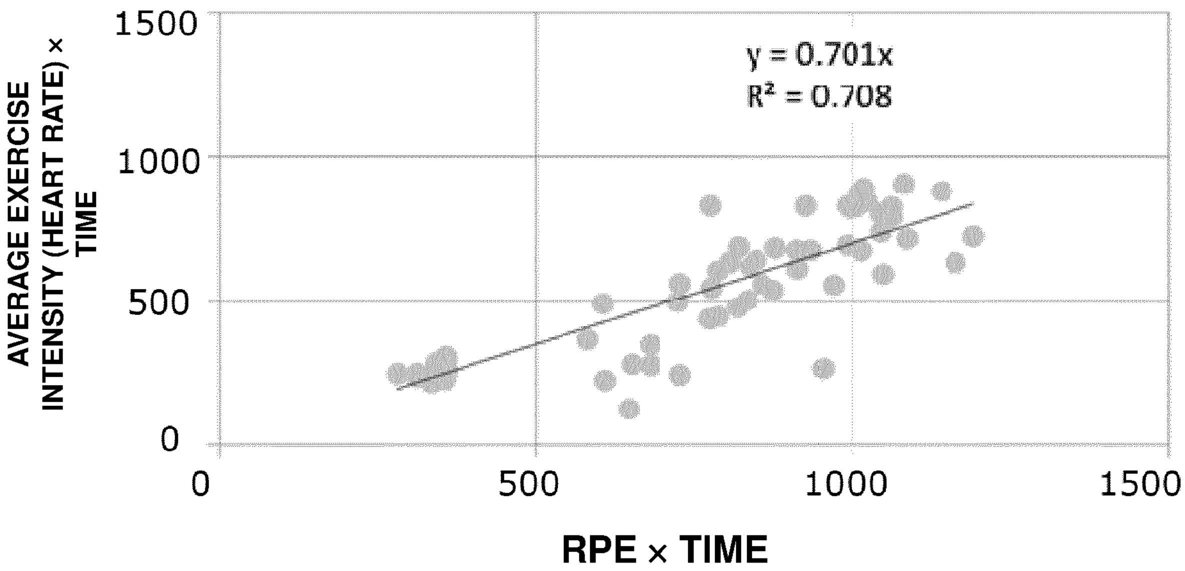
1500
1000
500
0
0
500
1000
1500
y = 0.701x
R² = 0.708
AVERAGE EXERCISE INTENSITY (HEART RATE) × TIME
RPE × TIME

# FIG.6

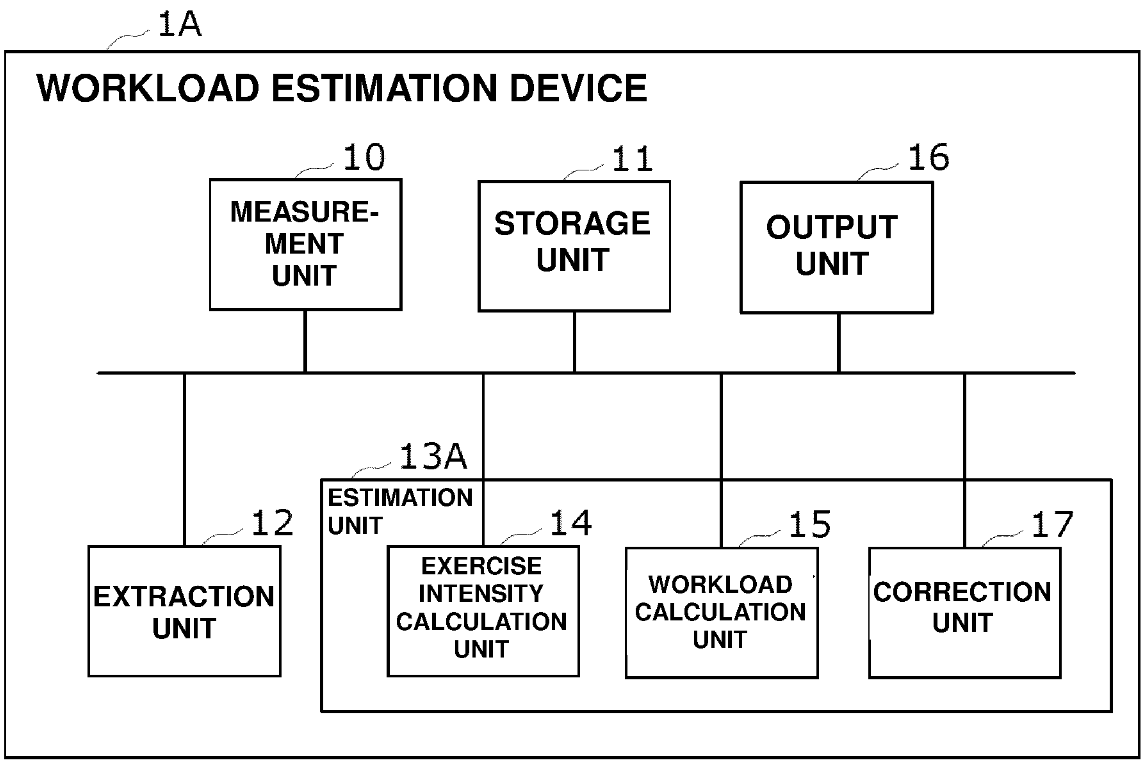
1A
WORKLOAD ESTIMATION DEVICE
10
MEASUREMENT UNIT
11
STORAGE UNIT
16
OUTPUT UNIT
13A
ESTIMATION UNIT
12
EXTRACTION UNIT
14
EXERCISE INTENSITY CALCULATION UNIT
15
WORKLOAD CALCULATION UNIT
17
CORRECTION UNIT

FIG.7

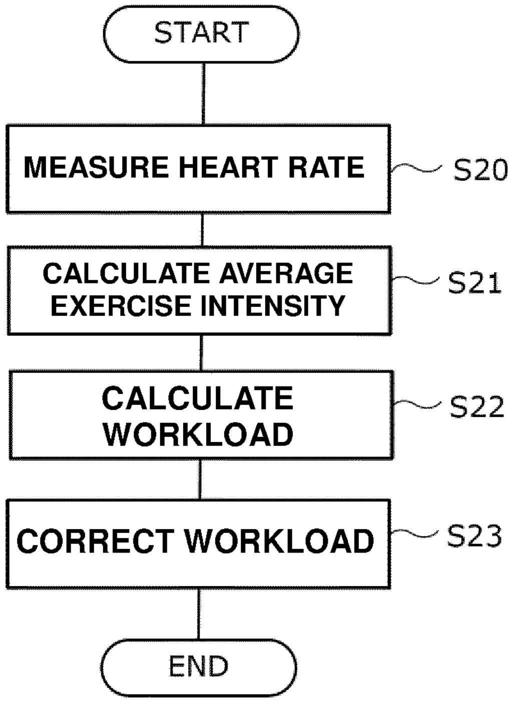
START
MEASURE HEART RATE
S20
CALCULATE AVERAGE EXERCISE INTENSITY
S21
CALCULATE WORKLOAD
S22
CORRECT WORKLOAD
S23
END

FIG.8

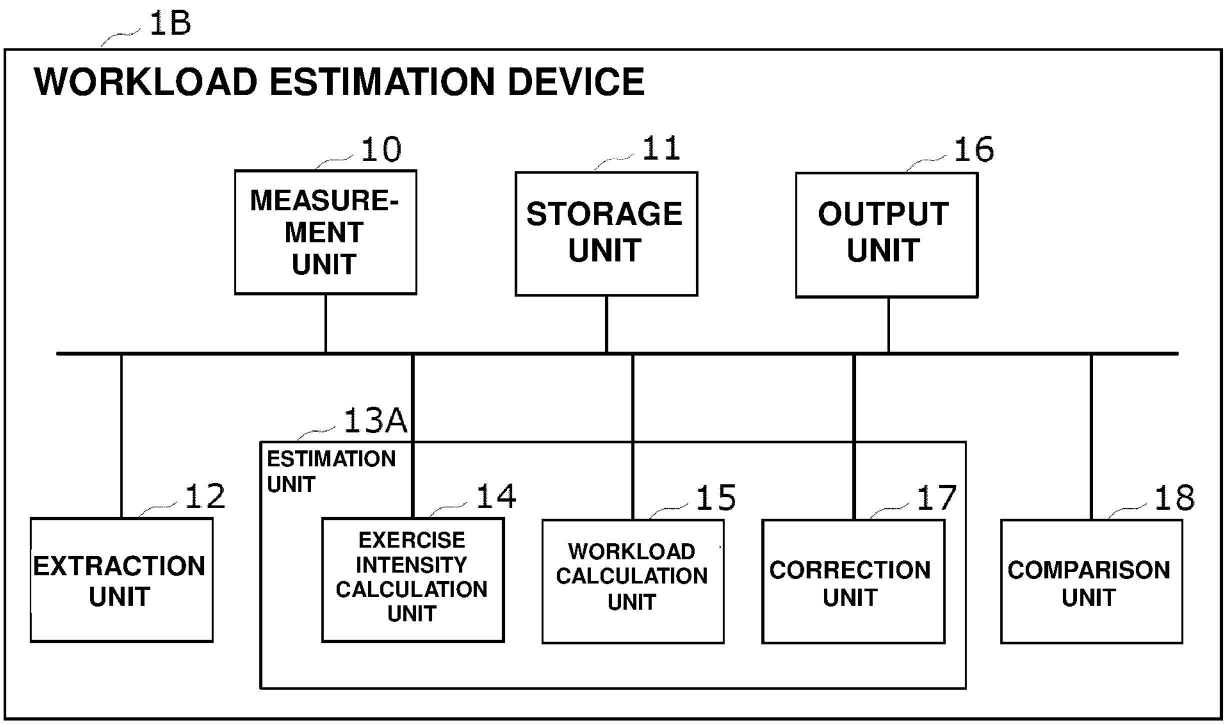
1B
WORKLOAD ESTIMATION DEVICE
10
MEASURE-MENT UNIT
11
STORAGE UNIT
16
OUTPUT UNIT
13A
ESTIMATION UNIT
12
EXTRACTION UNIT
14
EXERCISE INTENSITY CALCULATION UNIT
15
WORKLOAD CALCULATION UNIT
17
CORRECTION UNIT
18
COMPARISON UNIT

FIG.9

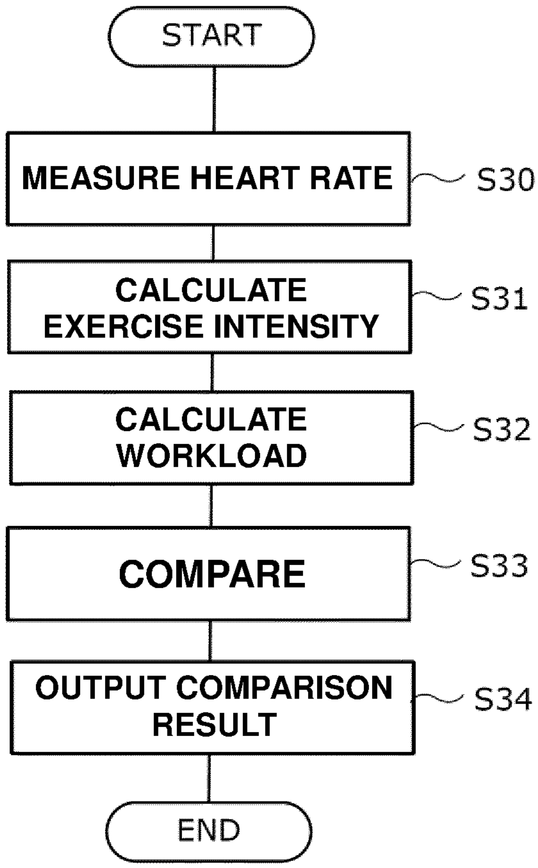
START
MEASURE HEART RATE
S30
CALCULATE EXERCISE INTENSITY
S31
CALCULATE WORKLOAD
S32
COMPARE
S33
OUTPUT COMPARISON RESULT
S34
END

FIG.10

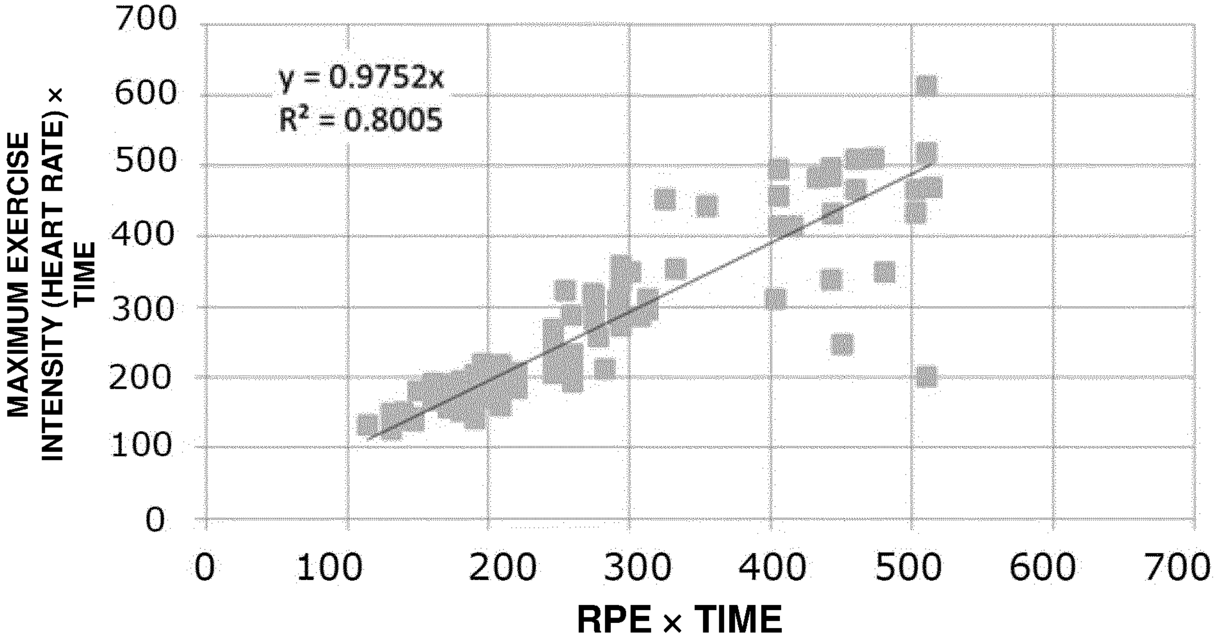
y = 0.9752x
R² = 0.8005
MAXIMUM EXERCISE INTENSITY (HEART RATE) × TIME
RPE × TIME
0
100
200
300
400
500
600
700

# FIG.11

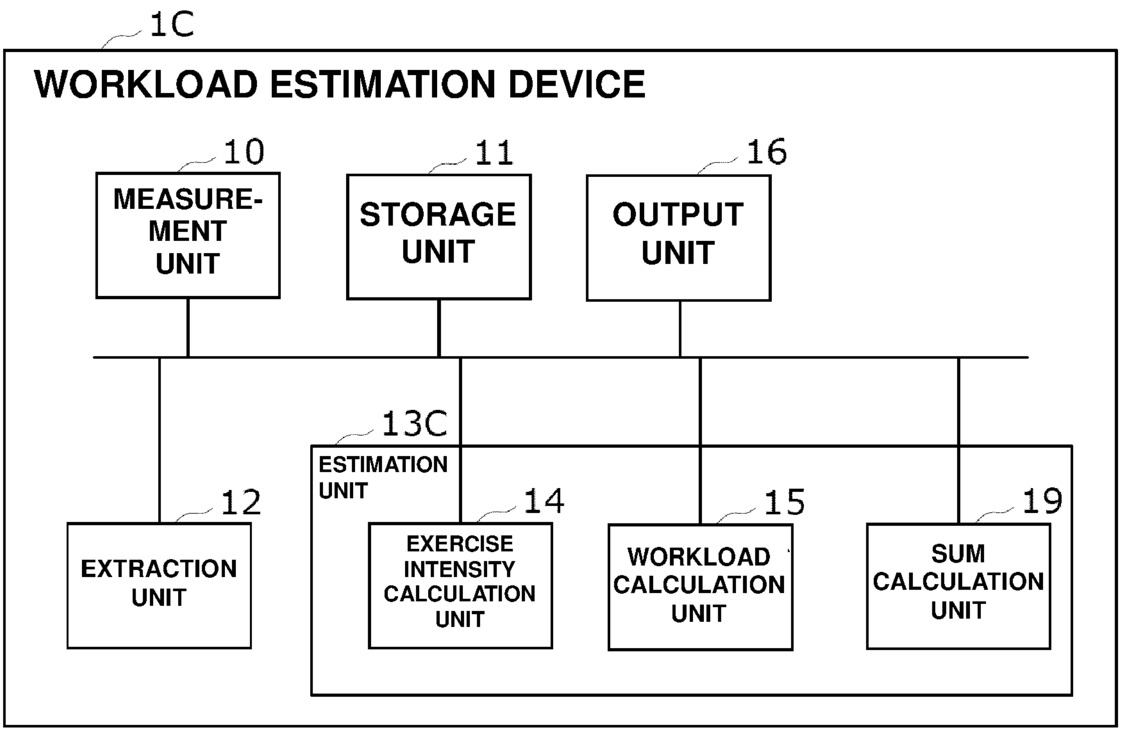
1C
WORKLOAD ESTIMATION DEVICE
10
MEASURE-
MENT
UNIT
11
STORAGE
UNIT
16
OUTPUT
UNIT
13C
ESTIMATION
UNIT
12
EXTRACTION
UNIT
14
EXERCISE
INTENSITY
CALCULATION
UNIT
15
WORKLOAD
CALCULATION
UNIT
19
SUM
CALCULATION
UNIT

# FIG.12

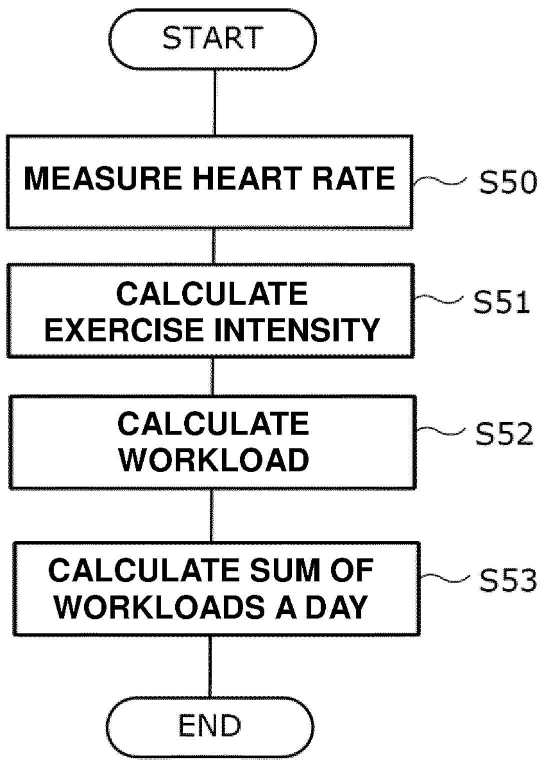
START
MEASURE HEART RATE
S50
CALCULATE
EXERCISE INTENSITY
S51
CALCULATE
WORKLOAD
S52
CALCULATE SUM OF
WORKLOADS A DAY
S53
END

FIG.13

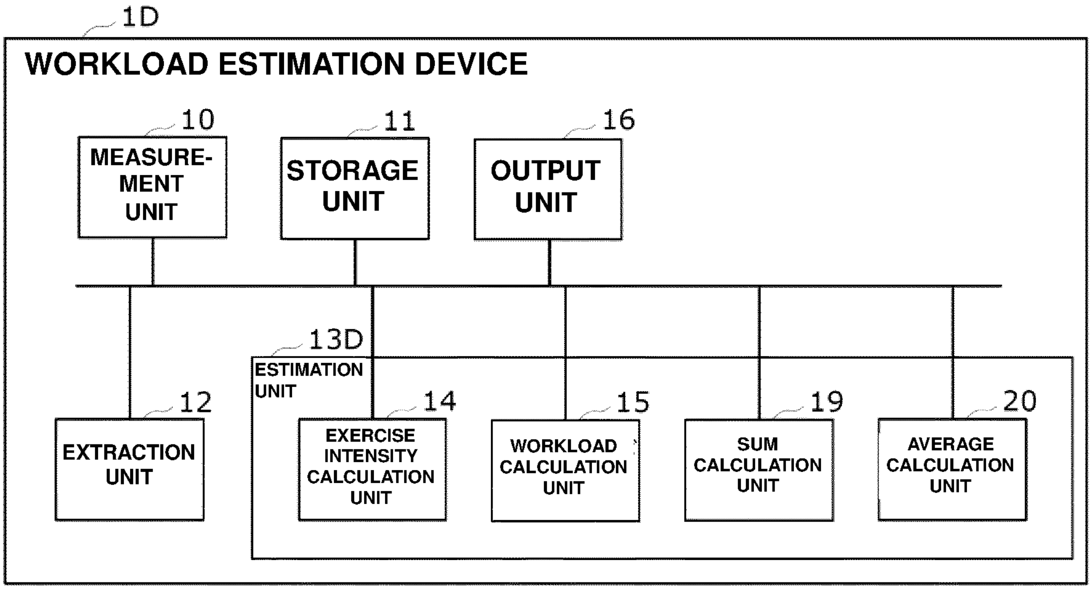
1D
WORKLOAD ESTIMATION DEVICE
10
MEASURE-MENT UNIT
11
STORAGE UNIT
16
OUTPUT UNIT
13D
ESTIMATION UNIT
12
EXTRACTION UNIT
14
EXERCISE INTENSITY CALCULATION UNIT
15
WORKLOAD CALCULATION UNIT
19
SUM CALCULATION UNIT
20
AVERAGE CALCULATION UNIT

FIG.14

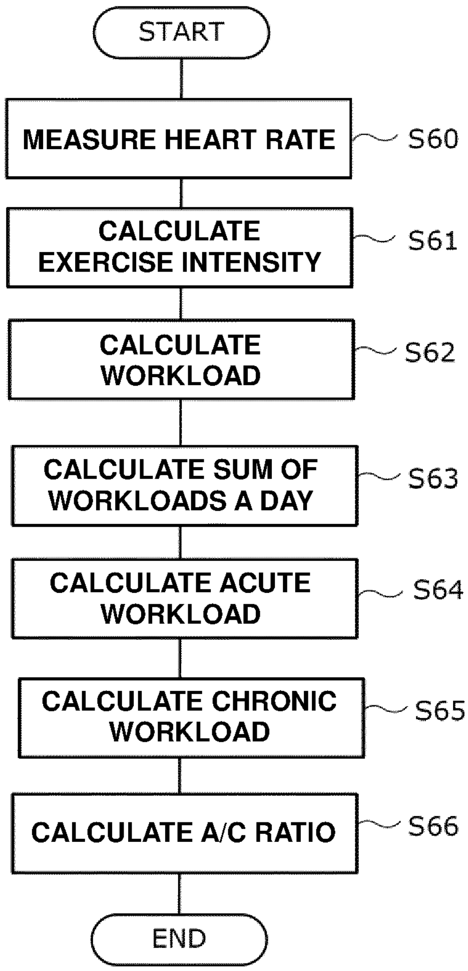
START
MEASURE HEART RATE
S60
CALCULATE EXERCISE INTENSITY
S61
CALCULATE WORKLOAD
S62
CALCULATE SUM OF WORKLOADS A DAY
S63
CALCULATE ACUTE WORKLOAD
S64
CALCULATE CHRONIC WORKLOAD
S65
CALCULATE A/C RATIO
S66
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090287103 A1
- CN 106730763
- US 20160354636 A1
- US 20160213979 A1

### Non-patent literature cited in the description

- **GABBETT, TIM J.** The training-injury prevention paradox: should athletes be training smarter and harder?. *Br J Sports Med,* 2016
- **JINHUA et al.** *Selection of Suitable Maximum-heart-rate Formulas for Use with Karvonen Formulate to Calculate exercise Intensity*